**Europäisches Patentamt**

(19) **European Patent Office**

**Office européen des brevets**

(11) Numéro de publication : **0 334 762 B1**

# (12) FASCICULE DE BREVET EUROPEEN

(45) Date de publication du fascicule du brevet :
**14.08.91 Bulletin 91/33**

(51) Int. Cl.⁵ : **G06F 15/62**, **G01N 23/04**, **H05G 1/26**

(21) Numéro de dépôt : **89400824.2**

(22) Date de dépôt : **23.03.89**

(54) Procédé et système d'étalonnage d'un scanner à rayons X en utilisant un seul étalon non circulaire.

(30) Priorité : **25.03.88 FR 8803583**

(43) Date de publication de la demande :
**27.09.89 Bulletin 89/39**

(45) Mention de la délivrance du brevet :
**14.08.91 Bulletin 91/33**

(84) Etats contractants désignés :
**DE ES GB IT NL**

(56) Documents cités :
**EP-A- 0 107 253**
**EP-A- 0 154 429**
**EP-A- 0 216 507**
**EP-A- 0 218 367**
**EP-A- 0 239 647**
**US-A- 4 352 020**

(73) Titulaire : **GENERAL ELECTRIC CGR S.A.**
**100, rue Camille-Desmoulins**
**F-92130 Issy les Moulineaux (FR)**

(72) Inventeur : **Feldman, Andrei**
**Cabinet Ballot-Schmit 84 Avenue Kléber**
**F-75116 Paris (FR)**
Inventeur : **Cornuejols, Dominique**
**Cabinet Ballot-Schmit 84 Avenue Kléber**
**F-75116 Paris (FR)**

(74) Mandataire : **Ballot, Paul Denis Jacques**
**Cabinet Ballot-Schmit 7, rue le Sueur**
**F-75116 Paris (FR)**

EP 0 334 762 B1

**Description**

L'invention concerne les scanners à rayons X et plus particulièrement un procédé d'étalonnage d'appareils de ce type qui utilise un seul étalon non circulaire et un système de mise en oeuvre dudit procédé.

Pour examiner un patient, on utilise de plus en plus des appareils à rayons X appelés "scanners" qui réalisent des images de coupes transversales du patient. Ces appareils sont basés sur le phénomène physique d'absorption des rayons X par le corps humain. Cette absorption est directement liée à la distance parcourue x des rayons X dans le corps selon la formule :

$$I = I_o e^{-bx}$$

formule dans laquelle :

$I_o$ est l'intensité du rayonnement entrant dans le corps humain

I est l'intensité du rayonnement sortant du corps humain,

b est un coefficient d'atténuation qui dépend du corps traversé.

Dans une échelle de mesure logarithmique, l'atténuation $I/I_o$ est égale à bx c'est-à-dire qu'elle est proportionnelle à la distance x.

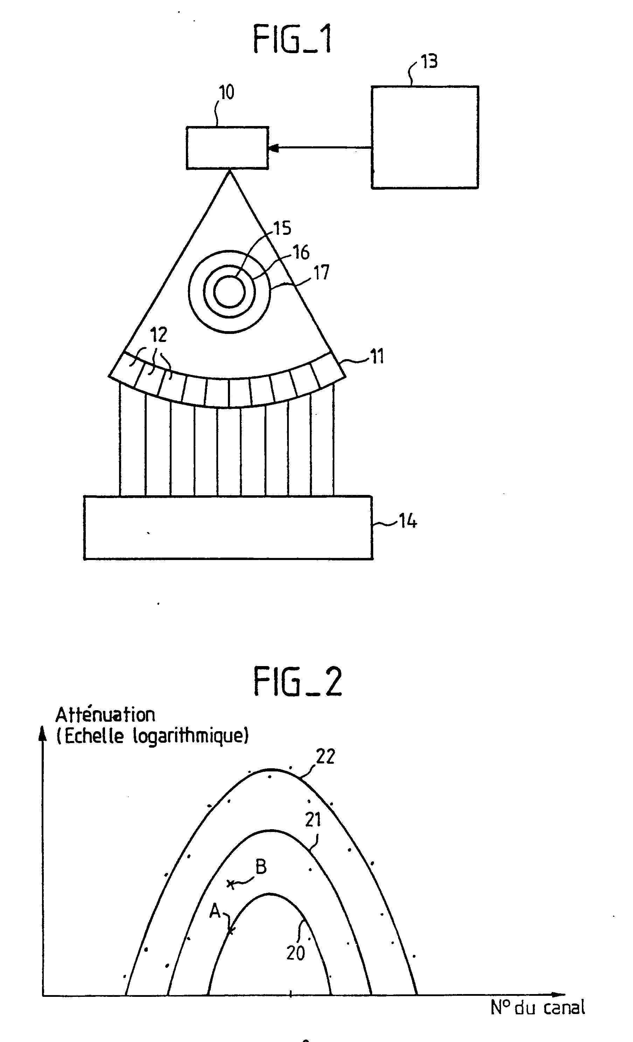

Ces appareils sont constitués essentiellement, comme le montre la figure 1, d'une source de rayons X 10 associée à un dispositif de détection 11, ces deux éléments étant disposés l'un par rapport à l'autre dans une relation géométrique fixe de manière à pouvoir intercaler entre eux le corps à examiner. En outre, ils sont supportés par une structure (non représentée) qui peut tourner autour du corps à examiner de manière à irradier le corps suivant des angles différents. La source à rayons X, qui est commandée par un dispositif 13, émet ses rayons suivant un secteur angulaire qui a une largeur suffisante pour illuminer toute la section transversale du corps. Le dispositif de détection 11 a la forme d'un secteur annulaire dont la longueur est adaptée à la largeur du faisceau de rayons X et est constitué d'un grand nombre de détecteurs élémentaires 12 juxtaposés les uns à côté des autres.

Pour obtenir une image de la section transversale du corps humain traversé par le faisceau de rayons X on fait tourner la structure de support de la source 10 et du dispositif de détection 11 autour du corps et on mesure les signaux de sortie des détecteurs élémentaires 12 pour les traiter de manière appropriée selon des procédés connus afin d'en tirer une image représentative de la section transversale. Pour ce traitement, les détecteurs élémentaires 12, également appelés canaux, sont connectés à un dispositif électronique 14 qui effectue en premier lieu un calcul du logarithme des signaux reçus de manière à obtenir un signal dont l'amplitude est proportionnelle à l'atténuation des rayons X.

Par suite de l'effet de différents phénomènes qui ne seront pas expliqués ici, l'amplitude de ce signal pour chaque détecteur élémentaire ou canal n'est pas proportionnelle à l'atténuation effectivement subie. En conséquence, pour remédier à cet inconvénient, il a été imaginé divers procédés qui consistent par exemple à relever les signaux de sortie des canaux en présence de corps dont on connaît les dimensions et le coefficient d'absorption, de manière à calculer les atténuations (calculs de logarithmes) et à comparer ces atténuations mesurées à des valeurs calculées en fonction des dimensions et du coefficient d'absorption du corps ou étalon. Ces comparaisons permettent de déduire une loi de correspondance ou une loi modificatrice entre les valeurs mesurées et les valeurs que l'on devrait obtenir. Cette loi peut être sous la forme de fichiers de correspondance ou de formules mathématiques traduisant cette correspondance pour chaque canal de détection.

Les étalons qui sont utilisés pour effectuer ces mesures dites d'étalonnage sont par exemple des cales d'épaisseurs différentes qui sont introduites à proximité de la source de rayons X, ce qui implique des manipulations au niveau de la source pour introduire et retirer ces cales. En outre, la forme de ces cales et leur position sont éloignées de la forme et de la position du corps du malade à examiner, ce qui augmente la non-linéarité du système.

Dans le brevet USP N° 4352020, il est proposé d'utiliser des cales circulaires 15 à 17, de différents diamètres, qui sont disposées au centre de rotation de la structure du support. Ceci permet de se rapprocher des conditions des mesures qui seront effectuées sur le corps à examiner. Ce brevet propose également d'utiliser un étalon en forme de cône à section circulaire qui est déplacé transversalement par rapport au faisceau de manière à obtenir différentes longueurs d'atténuation. Avec les étalons décrits, les mesures sont effectuées pour une position déterminée de la structure de support et par étalon.

La figure 2 montre l'allure de trois courbes de réponse 20, 21 et 22 de l'atténuation en fonction de la position des canaux dans le cas de mesures sur trois étalons de forme circulaire. Les valeurs mesurées sont représentées par les pointillés et varient autour d'une valeur moyenne qui représente la valeur théorique dans un système linéaire. Ces courbes peuvent être utilisées de la manière suivante ; lorsque le signal mesuré correspond à un point A, on en déduira que le signal linéaire est le point A' de la courbe moyenne 20. Lorsque le signal mesuré correspond à un point B situé entre les courbes 20 et 21, on en déduira le signal linéaire par interpolation entre les courbes 20 et 21. Cette interpolation peut être calculée suivant une loi linéaire ou plus généralement une loi polynomiale.

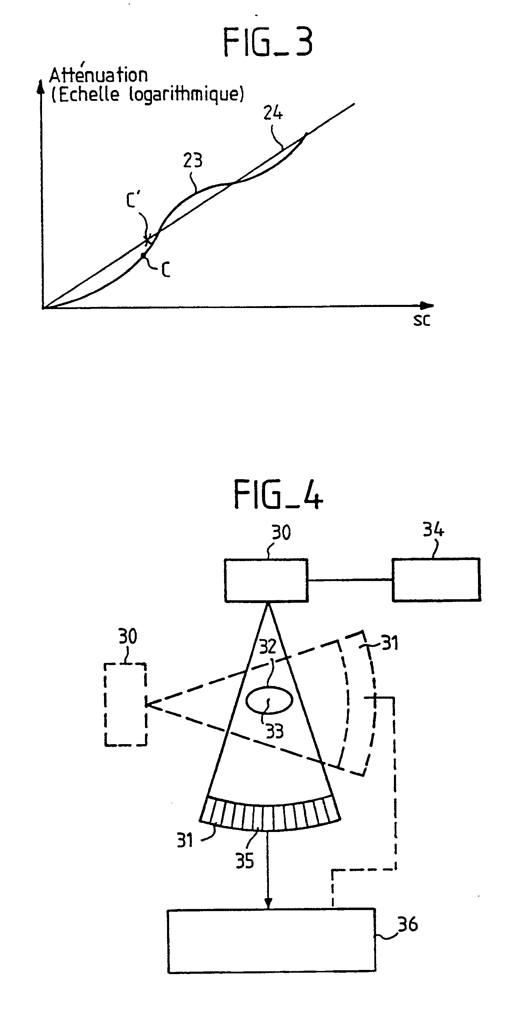

Les courbes 23 et 24 de la figure 3 montrent sous une autre forme le principe de l'étalonnage au niveau d'un canal. Il s'agit de courbes décrivant dans un canal donné l'atténuation en fonction de l'apaisseur x pour des valeurs mesurées (courbe 23) et pour des valeurs calculées (droite 24) : en fait, les valeurs mesurées donnent des points, points que l'on relie entre eux selon une loi choisie : linéaire ou polynomiale, de manière à obtenir une courbe continue. Lorsqu'on mesure une atténuation, celà correspond par exemple au point C de la courbe 23 et on en déduit la valeur linéaire correspondant au point C' de la courbe 24.

Le brevet américain précité décrit un appareil dans lequel la correspondance entre les valeurs mesurées et les valeurs réelles d'atténuation est effectuée par un système de fichiers créés pendant l'opération d'étalonnage. En ce qui concerne l'interpolation, le brevet propose des interpolations linéaires, cubiques et biquadratiques mais seule l'interpolation linéaire est décrite de manière détaillée.

Les procédés d'étalonnage qui ont été succinctement décrits ci-dessus présentent l'inconvénient majeur de mettre en oeuvre plusieurs étalons, ce qui conduit à de nombreuses manipulations ; en outre ces manipulations doivent être précises, notamment dans le cas d'étalons circulaires dont les différents centres doivent coïncider avec le centre de rotation de la structure. Il est à noter que le brevet américain propose d'utiliser un étalon unique qui aurait la forme donnée par la figure 12 dudit brevet, et de faire tourner la structure autour de cet étalon, ce qui conduit à obtenir des trajets d'absorption de longueur différente selon la position angulaire de la structure. Cependant, une telle manière de faire n'est que citée et n'indique pas le procédé ni les moyens de mettre en oeuvre dans ce cas. Un but de la présente invention est de mettre en oeuvre un procédé d'étalonnage qui utilise un étalon unique ayant une forme autre que circulaire, notamment une forme elliptique ou une forme qui serait symétrique par rapport à deux axes coplanaires.

Un autre but de la présente invention est de réaliser un appareil d'étalonnage pour mettre en oeuvre ledit procédé.

L'invention se rapporte à un procédé d'étalonnage d'un scanner à rayons X tel que revendiqué dans la revendication 1.

L'invention se rapporte également à un appareil d'étalonnage d'un scanner à rayons X tel que revendiqué dans la revendication 7.

D'autres caractéristiques et avantages de la présente invention apparaîtront à la lecture de la description suivante d'un exemple particulier de réalisation, ladite description étant faite en relation avec les dessins joints dans lesquels :

— la figure 1 est un schéma de principe d'un scanner à rayons X dans lequel l'étalonnage est obtenu à l'aide d'étalons circulaires,

— la figure 2 est un diagramme montrant différentes courbes d'atténuation en fonction de la position des détecteurs ou canaux et du diamètre de l'étalon ;

— la figure 3 est un diagramme montrant les courbes d'atténuation théorique et mesurée pour un canal déterminé en fonction du trajet d'absorption ;

— la figure 4 est un schéma de principe d'un scanner à rayons X dans lequel l'étalonnage est obtenu selon des caractéristiques de l'invention ;

— la figure 5 est un diagramme montrant différentes courbes d'atténuation en fonction de la position du détecteur ou canal et de la position de la source à rayons X et des détecteurs par rapport à l'étalon unique et,

— la figure 6 est un schéma fonctionnel d'un système de traitement des signaux de sortie des détecteurs.

Les figures 1, 2 et 3 qui ont permis de définir l'art antérieur dans le préambule ne seront pas décrites à nouveau.

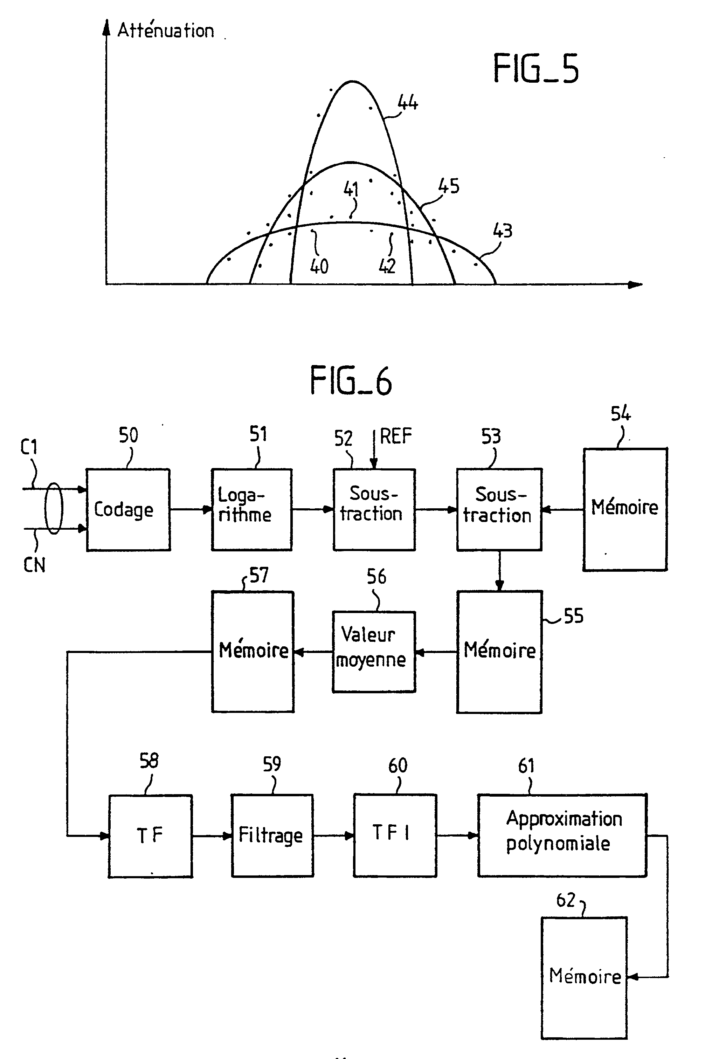

Le schéma de la figure 4 est analogue à celui de la figure 1 en ce sens qu'il montre un scanner à rayons X qui comprend une source à rayons X 30 et un dispositif de détection 31 à plusieurs détecteurs ou canaux 35, entre lesquels est interposé, en fonctionnement normal, le corps d'un patient ou un étalon 32 de forme élliptique lors des opérations d'étalonnage. Bien que non représentée, une structure est prévue pour supporter la source 30 et le dispositif 31 et faire tourner l'ensemble autour du corps du patient ou de l'étalon, l'axe de rotation étant matérialisé par le point 33 situé dans l'étalon mais pas nécessairement en son centre géométrique. La source à rayons X 30 est commandée par un dispositif de commande 34 tandis que les différents détecteurs ou canaux 35 du dispositif 31 sont connectés à un système électronique 36 qui sera décrit en relation avec la figure 6.

Le procédé d'étalonnage selon l'invention consiste à disposer la structure de support dans différentes positions angulaires dites principales, autour de l'étalon et à effectuer, à chaque position angulaire et de part et d'autre de cette position, une série de mesures de l'atténuation. Par exemple, on peut choisir seize positions angulaires principales régulièrement espacées de 22° 30' et effectuer par exemple dix mesures élémentaires sur tous les canaux de détection autour de chaque position angulaire principale. A titre d'exemple, si le nombre de détecteurs est de 1024, le nombre total de vues sera de 16 × 10 : 160 et le nombre total de mesures sera de 1024 × 160 : 163840. A titre d'exemple, chaque vue autour d'une position angulaire principale est séparée d'un angle d'environ un tiers de degré.

Ainsi donc, pour chaque position angulaire principale, il y a dix vues sur chacun des 1024 canaux, c'est-à-dire dix mesures par canal sur un secteur angulaire de trois degrés environ, et chaque mesure correspond à un trajet légèrement différent dans l'étalon et donc à une atténuation légèrement différente. Selon l'invention, dans chaque canal, ces dix mesures sont utilisées pour calculer une moyenne ce qui permet d'améliorer le rapport signal/bruit par un coefficient $\sqrt{10}$. Ces valeurs moyennes au nombre de seize permettent de tracer, pour chaque position angulaire principale, une courbe de variation de l'atténuation en fonction de la position des canaux. En fait, ce nombre de courbes peut être ramené à quatre car les seize positions angulaires principales se réduisent à quatre si l'on tient compte des symétries entre les positions angulaires principales et celles de l'étalon.

Ces courbes de variation sont montrées sur la figure 5 pour trois des quatre positions angulaires principales après prise en compte des symétries. Sur cette figure, les points 40, 41 ou 42 représentent, les valeurs moyennes d'atténuation pour certains des 1024 canaux, et la courbe 43 résulte d'une opération de lissage conformément à la présente invention, opération qui sera décrite ci-après en relation avec la figure 6.

La courbe 43 correspond à la position angulaire de la figure 4, c'est-à-dire que l'étalon elliptique 32 est irradié suivant le grand axe de l'ellipse. La courbe 44 correspond à la position angulaire représentée en pointillés sur la figure 4, c'est-à-dire que l'étalon 32 est irradié suivant le petit axe de l'ellipse. Enfin, la courbe 45 correspond à une position intermédiaire entre les deux précédentes.

Par ailleurs, comme indiqué ci-dessus les seize positions angulaires principales sont symétriques deux à deux, les deux positions symétriques étant séparées deux à deux d'un angle de 180°. Ces deux positions symétriques conduisent à des courbes d'atténuation identiques et il est suffisant de n'en retenir qu'une seule. En fait, selon l'invention, il est proposé de combiner les mesures provenant de ces deux positions symétriques en en effectuant la moyenne de la manière qui sera expliquée plus en détail en relation avec la figure 6. Le rapport signal/bruit est alors amélioré par un facteur $\sqrt{2}$ soit $\sqrt{20}$ au total.

En outre, si un étalon de forme symétrique par rapport à deux axes, par exemple orthogonaux, est utilisé, on peut grouper les mesures correspondant à des positions symétriques par rapport à ces axes. Il en résulte que les seize positions principales, qui ont été ramenées à huit par la première symétrie peuvent à nouveau être groupées deux par deux en en effectuant la moyenne, ce qui permet d'aboutir à quatre positions angulaires principales. Le rapport signal/bruit est à nouveau amélioré de $\sqrt{2}$, soit $\sqrt{40}$ au total. Les aspects principaux du procédé d'étalonnage selon l'invention sont :

— l'utilisation d'un étalon non circulaire ;

— la mesure des signaux de sortie des N canaux pour positions angulaires principales P de la structure source-détecteur et pour n positions élémentaires ou vues proches les unes des autres autour de chaque position angulaire principale ;

— le calcul de la moyenne des n mesures pour chaque canal et pour chacune des P positions angulaires principales ;

— le lissage des valeurs moyennes d'un canal au suivant pour chaque position angulaire principale de manière à obtenir une courbe de réponse dans laquelle les composantes haute fréquence ont été éliminées.

Dans le cas de positions angulaires principales espacées régulièrement et en nombre pair il est prévu un calcul de la valeur moyenne des mesures correspondant à des positions principales espacées de 180°. En outre, si l'étalon a une forme symétrique par rapport à deux axes, le calcul de la valeur moyenne est effectué également sur les mesures obtenues pour des positions symétriques par rapport auxdits axes.

Pour mettre en oeuvre le procédé, il est proposé de réaliser un système dont le schéma fonctionnel est donné par la figure 6. Sur cette figure, les signaux fournis par les 1024 détecteurs sont appliqués à un circuit de codage 50 qui fournit des valeurs ou codes numériques. Ce sont sur ces codes que seront effectuées toutes les opérations qui vont être décrites ci-après, ces opérations pouvant être réalisées par un calculateur convenablement programmé.

Les N codes résultant d'une mesure à une position élémentaire ou vue sont appliqués à un circuit de calcul de logarithme 51. Les valeurs logarithmiques sont soustraites dans un circuit 52 à une valeur logarithmique de référence REF fournie par un détecteur appelé moniteur qui reçoit directement le rayonnement X sans atténuation. Cette différence donne donc la valeur de l'atténuation introduite par l'étalon par rapport à un trajet dans l'air. Ces N valeurs différentielles, correspondant chacune à un canal, sont ensuite soustraites dans un circuit 53 à N valeurs logarithmiques d'atténuation mesurées en l'absence d'étalon, c'est-à-dire selon des trajets dans l'air. Ces valeurs ont été mesurées et calculées préalablement à ce procédé d'étalonnage et sont utilisées ensuite, comme il est connu, lors des mesures sur le patient. Elles sont pour cette raison enregistrées dans une mémoire 54. Cette deuxième opération de soustraction permet de tenir compte d'une partie des disparités entre les canaux et donc de supprimer leur influence d'un canal au suivant. Les N valeurs résultant de cette deuxième soustraction sont enregistrées dans une mémoire 55 qui est prévue pour recevoir les $N \cdot P \cdot n \cdot$ codes qui résulteront des n mesures effectuées (ou n vues prises) à n positions élémentaires autour d'une position angulaire principale. Ce sont les n · N · codes qu'est effectué le calcul de la moyenne sur chaque canal par l'intermédiaire d'un circuit 56. Les codes des N valeurs moyennes sont enregistrés dans une mémoire 57. On comprend que la mémoire 55 et le circuit de calcul 56 peuvent être réalisés sous la forme d'un circuit additionneur qui réaliserait neuf opérations successives d'addition. Pour bénéficier des effets de la symétrie des P positions angulaires principales et de l'étalon, plusieurs manières de faire peuvent être mises en oeuvre. L'une d'entre elles consiste à effectuer successivement les mesures pour quatre positions angulaires principales symétriques et à effectuer la moyenne pour un canal, non pas sur dix valeurs mais sur quarante valeurs. Ceci implique une mémoire 55 prévue pour recevoir 40.N codes. Une autre manière consiste à utiliser P mémoires 57, une par position angulaire principale, et à effectuer un deuxième calcul de la moyenne sur les valeurs contenues dans les quatre mémoires correspondant aux quatre positions angulaires principales symétriques.

Après ces opérations de calcul de la moyenne qui ont pour but principal de réduire le bruit et donc d'améliorer le rapport signal/bruit, interviennent les opérations de lissage qui sont de deux ordres. D'abord une opération d'élimination des hautes fréquences dans des circuits 58, 59 et 60 et une opération d'approximation polynomiale dans un circuit 61. L'opération d'élimination des hautes fréquences comporte d'abord une transformée de Fourier dans le circuit 58 pour obtenir le spectre du signal sur les N canaux, puis un filtrage passe-bas dans le circuit 59 qui élimine les composantes haute fréquence, enfin une transformée de Fourier inverse de manière à revenir au signal initial mais sans composante haute fréquence.

L'opération d'approximation polynomiale dans le circuit 61 peut être limitée à la partie centrale des courbes 43, 44 et 45 de la figure 5. Elle est effectuée par tous procédés et moyens connus. Les résultats de cette approximation polynomiale sont enregistrés dans une mémoire 62 qui peut contenir les valeurs numériques des P, P/2 ou P/4 courbes lissées selon que l'on tient compte ou non des symétries mentionnées ci-dessus, les courbes étant calculées l'une d'après l'autre.

Les valeurs enregistrées dans la mémoire 62 à la fin des opérations pour P positions angulaires principales constituent des valeurs d'étalonnage qui sont utilisées de manière connue, lors des opérations de mesure sur le patient, pour calculer la valeur réelle de l'atténuation à partir de la valeur mesurée.

Chaque courbe ainsi lissée de la mémoire 62, correspond à une courbe non lissée de la mémoire 57 et la différence entre les valeurs de ces deux courbes pour un même canal permet de calculer une valeur d'écart. Ainsi, lorsque pour ce même canal, la différence entre la valeur mesurée avec le patient et la valeur lissée est comprise dans cette valeur d'écart, on en déduit que la valeur réelle est celle de la courbe lissée. Lorsque cette différence dépasse cette valeur d'écart, il est nécessaire d'effectuer un calcul d'interpolation de type linéaire ou polynomial pour déterminer la valeur réelle comprise entre deux courbes lissées.

Le système de mise en oeuvre du procédé a été décrit sous la forme de circuits fonctionnels mais il est clair qu'il peut être réalisé pour un calculateur qui réaliserait par programme les différentes opérations décrites.

## Revendications

1. Procédé d'étalonnage d'un scanner à rayons X qui comporte une source (30) de rayonnement X et un dispositif (31) de détection à N canaux portés par une structure qui tourne autour d'un axe (33), en utilisant un seul étalon (32) de forme autre que circulaire, caractérisé en ce qu'il comprend les opérations suivantes :

a) la mise en place de l'étalon entre la source de rayonnement X et le dispositif de détection ;

b) le calcul (51-55) de N × P × n signaux atténuation à partir des signaux obtenus dans les N canaux du dispositif de détection pour P positions angulaires principales de la structure tournante et pour n positions élémentaires ou vues autour de chaque position angulaire principale ;

c) le calcul (56), pour chaque canal et pour chacune des P positions angulaires principales, d'un signal de moyenne des n signaux d'atténuation correspondant aux n positions élémentaires autour de chaque position angulaire principale ;

d) le lissage (58-61) des N signaux de moyenne d'un canal au suivant pour chacune des P positions angulaires principales de manière à obtenir une courbe de réponse (43, 44, 45) de l'atténuation en fonction de la position du canal dans laquelle les composantes haute fréquence ont été éliminées, ladite courbe de réponse ainsi obtenue étant la courbe réelle de variation de l'atténuation introduite par l'étalon en fonction de la position respective des N canaux.

2. Procédé d'étalonnage selon la revendication 1, caractérisé en ce que l'opération de lissage comprend une opération de filtrage (59).

3. Procédé d'étalonnage selon la revendication 2 caractérisé en ce que l'opération de lissage comprend, en outre, une opération d'approximation polynomiale (61) qui est effectuée sur la courbe résultant du filtrage.

4. Procédé d'étalonnage selon la revendication 2 ou 3, caractérisé en ce que l'opération de filtrage consiste à réaliser la transformée de Fourier (58) des N valeurs moyennes correspondant à une des P positions angulaires principales, à filtrer (59) le signal obtenu pour éliminer les composantes haute fréquence et à réaliser ensuite la transformée de Fourier inverse (60).

5. Procéde selon l'une quelconque des revendications précédentes, caractérisé en ce que les P positions angulaires principales sont en nombre pair, et sont choisies de manière à être symétriques deux à deux par rapport au centre de rotation et en ce que les valeurs des atténuations correspondant à des positions élémentaires symétriques sont moyennées.

6. Procédé selon l'une quelconque des revendications précédentes, caractérisé en ce que l'étalon a une forme qui est symétrique par rapport à deux axes et en ce que les signaux d'atténuation correspondant à des positions symétriques de l'étalon par rapport auxdits axes sont moyennées.

7. Appareil d'étalonnage d'un scanner à rayons X qui comporte une source (30) de rayonnement X et un dispositif (31) de détection à N canaux portés par une structure qui tourne autour d'un axe (33) en utilisant un seul étalon (32) de forme autre que circulaire, caractérisé en ce qu'il comprend :

   — des moyens de mise en place de l'étalon entre la source de rayonnement X et le dispositif de détection ;

   — des premiers moyens (51) pour calculer le logarithme des N signaux fournis par les N détecteurs,

   — des deuxièmes moyens (52-55) pour calculer les N x P x n signaux d'atténuation à partir des signaux reçus des N premiers moyens pour P positions angulaires principales de la structure tournante et pour n positions élémentaires ou vues autour de chaque position angulaire principale ;

   — des troisièmes moyens (56) pour calculer, pour chaque canal et pour chacune des positions angulaires principales, la valeur moyenne des n signaux d'atténuation correspondant aux n positions élémentaires autour de chaque position angulaire principale,

   — des quatrièmes moyens (58) pour calculer des signaux représentatifs de la transformée de Fourier des N signaux d'atténuation moyenne correspondant à une position angulaire principale,

   — des cinquièmes moyens (59) pour éliminer les composantes haute fréquence dans les signaux de spectre résultant de la transformée de Fourier,

   — des sixièmes moyens (60) pour calculer la transformée de Fourier inverse des signaux basse fréquence du spectre,

   — des septièmes moyens (61) pour calculer une approximation polynomiale de la courbe (20, 21, 22) que forment les signaux résultant de la transformée de Fourier inverse,

   — et des moyens (62) pour garder en mémoire les valeurs représentant l'approximation polynomiale de chaque courbe de réponse relative à une position angulaire principale.

## Patentansprüche

1. Eichungsverfahren für einen Röntgenstrahl-Scanner mit einer Röntgenstrahlquelle (30) und einer N-Kanal- Detektorvorrichtung (31), die von einem Aufbau, der sich um eine Achse (33) dreht, gehalten wird, wobei ein einziger Eichkörper (32), der nicht kreisförmig ist, verwendet wird, dadurch gekennzeichnet, daß es die folgenden Operationen umfaßt :

   a) Einsetzen des Eichkörpers zwischen die Röntgenstrahlquelle und die Detektorvorrichtung ;

   b) Berechnen (51-55) von N · P · n Dämpfungssignalen anhand der Signale, die in den N Kanälen der Detektorvorrichtung für P Hauptwinkelpositionen des drehbaren Aufbaus und für n Elementarpositionen oder Meßrichtungen um jede Hauptwinkelposition erhalten werden ;

   c) Berechnen (56) eines Mittelwertsignals der den n Elementarpositionen um jede Hauptwinkelposition entsprechenden n Dämpfungssignale für jeden Kanal und für jede der P Hauptwinkelpositionen ;

   d) Glätten (58-61) der N Mittelwertsignale eines Kanals nach dem anderen für jede der P Hauptwinkelpositionen, derart, daß eine Dämpfungscharakteristik (43, 44, 45) in Abhängigkeit von der Position des Kanals erhalten wird, in der die Hochfrequenzkomponenten beseitigt sind, wobei die auf diese Weise erhaltene Charakteristik die tatsächliche Kurve der Dämpfungsvariation ist, die durch den Eichkörper in Abhängigkeit von der jeweiligen Position der N Kanäle eingebracht wird.

2. Eichungsverfahren gemäß Anspruch 1, dadurch gekennzeichnet, daß die Glättungsoperation eine Filterungsoperation (59) umfaßt.

3. Eichungsverfahren gemäß Anspruch 2, dadurch gekennzeichnet, daß die Glättungsoperation außerdem eine Polynomapproximations-Operation (61) umfaßt, die für die aus der Filterung sich ergebende Kurve ausgeführt wird.

4. Eichungsverfahren gemäß Anspruch 2 oder 3, dadurch gekennzeichnet, daß die Filterungsoperation in der Herstellung der Fourier-Transformierten (58) der einer der P Hauptwinkelpositionen entsprechenden N Mittelwerte, in der Filterung (59) des erhaltenen Signals zur Beseitigung der Hochfrequenzkomponenten und in der daran anschließenden Herstellung der inversen Fourier-Transformierten (60) besteht.

5. Verfahren gemäß einem der vorangehenden Ansprüche, dadurch gekennzeichnet, daß die P Hauptwinkelpositionen in gerader Anzahl vorhanden sind und so gewählt werden, daß sie paarweise in bezug auf das Drehzentrum symmetrisch sind und daß die den symmetrischen Elementarpositionen entsprechenden Dämpfungswerte gemittelt werden.

6. Verfahren gemäß einem der vorangehenden Ansprüche, dadurch gekennzeichnet, daß der Eichkörper eine Form besitzt, die in bezug auf zwei Achsen symmetrisch ist, und daß die Dämpfungssignale, die den in bezug auf diese Achsen symmetrischen Positionen des Eichkörpers entsprechen, gemittelt werden.

7. Eichungsgerät eines Röntgenstrahl-Scanners mit einer Röntgenstrahlquelle (30) und einer N-Kanal-Detektorvorrichtung (31), die von einem Aufbau, der sich um eine Achse (33) dreht, gehalten wird, wobei ein einziger Eichkörper (32), der nicht kreisförmig ist, verwendet wird, dadurch gekennzeichnet, daß es umfaßt :

— Mittel zum Einsetzen des Eichkörpers zwischen die Röntgenstrahlquelle und die Detektorvorrichtung ;

— erste Mittel (51) zum Berechnen des Logarithmus der von den N Detektoren gelieferten N Signale ;

— zweite Mittel (52-55) zum Berechnen der N · P · n Dämpfungssignale anhand der von den N ersten Mitteln empfangenen Signale für P Hauptwinkelpositionen des drehbaren Aufbaus und für n Elementarpositionen oder Meßrichtungen um jede Hauptwinkelposition ;

— dritte Mittel (56) zum Berechnen des Mittelwerts der den n Elementarpositionen um jede Hauptwinkelposition entsprechenden n Dämpfungssignale für jeden Kanal und für jede der Hauptwinkelpositionen ;

— vierte Mittel (58) zum Berechnen der Signale, die die Fourier-Transformierte der einer Hauptwinkelposition entsprechenden mittleren Dämpfungssignale darstellen ;

— fünfte Mittel (59) zum Beseitigen der Hochfrequenzkomponenten in den Signalen des aus der Fourier- Transformierten sich ergebenden Spektrums ;

— sechste Mittel (60) zum Berechnen der inversen Fourier- Transformierten der Niederfrequenzsignale des Spektrums ;

— siebte Mittel (61) zum Berechnen einer Polynomapproximation der Kurve (20, 21, 22), die von den aus der inversen Fourier- Transformierten sich ergebenden Signalen gebildet wird ;

— und Mittel (62) zum Speichern der Werte, die die Polynomapproximation einer jeden Charakteristik in bezug auf eine Hauptwinkelposition darstellen.

## Claims

1. A method for the calibration of an x-ray scanner which comprises a source (30) of x-rays and a device (31) for detection in N channels carried by a structure which turns around an axis (33) using a single standard (32) with a non-circular configuration, characterized in that it comprises the following operations :

a) the putting into position of the standard between the x-ray source and the detection device ;

b) the computation (51 to 55) of N · P · n attenuation signals starting with signals produced in the N channels of the detection device for P principal angular positions of the turning structure and for n elementary position or views around each principal angular position ;

c) the computation (56), for each channel and for each of the P principal angular positions, of a mean signal of the n attenuation signals corresponding to the n elementary positions around each principal angular position ;

d) the smoothing (58 to 61) of the N signals as a mean between one signal and the following one for each of the P principal angular positions in such a manner as to produce a response curve (43, 44 and 45) for the attenuation as a function of the position of the channel in which the high frequency components have been eliminated, the said response curve so produced being the real variation curve for the attenuation introduced by the standard as a function of the respective position of the N channels.

2. The calibration method as claimed in claim 1 characterized in that the smoothing operation comprises a filtering operation (59).

3. The calibration method as claimed in claim 2, characterized in that the smoothing operation furthermore comprises a polynomial approximation operation (61) which is performed on the curve resulting from such filtering.

4. The calibration method as claimed in claim 2 or claim 3, characterized in that the filtering operation consists of performing a Fourier transformation (58) on N mean values corresponding to one of the P principal angular positions, of filtering (59) the signal produced in order to eliminate the high frequency components and of then performing the inverse Fourier transformation (60).

5. The calibration method as claimed in any one of the preceding claims characterized in that the P principal angular positions are even in number and are selected in such a manner as to be symmetrical in pairs in relation to the center of rotation and in that the attenuation values corresponding to elementary symmetrical positions are averaged.

6. The calibration method as claimed in any one of the preceding claims, characterized in that the standard has a configuration, which is symmetrical in relation to two axes and in that the attenuation signals corresponding to symmetrical positions of the standard in relation to the said axes are averaged.

7. An apparatus for the calibration of an x-ray scanner, which comprises an x-ray source (30) and a detection device (31) with N channels carried by a structure which turns about an axis (33) using a single standard (32) with a non-circular form, characterized in that it comprises :

— means for the putting in place of the standard between the x-ray source and the detection device ;

— first means (51) in order to calculate the logarithm of the N signals supplied by the N detectors,

— second means (52 to 55) in order to compute the $N \cdot P \cdot n$ attenuation signals starting with the signal received from the N first means for P principal angular position of the turning structure and for n elementary positions or views around each principal angular position ;

— third means (56) in order to calculate, for each channel and each of the principal angular positions, the mean value of the n attenuation signal corresponding to the n elementy positions around each principal angular position,

— fourth means (58) in order to compute signals representative of the Fourier transformation of the N signals with mean attenuation corresponding to a principal angular position,

— fifth means (59) in order to eliminate the high frequency components in the spectrum signal resulting from the Fourier transformation,

— sixth means (60) in order to calculate the inverse Fourier transformation of the low frequency signals of the spectrum,

— seventh means (61) in order to calculate a polynomial approximation of the curve (20, 21 and 22) which constitutes the signal resulting from the inverse Fourier transformation, and

— means (62) in order to store in a memory the values representative of the polynomial approximation for each response curve relating to one principal angular position.

# FIG_1

# FIG_2

# FIG_3

Atténuation
(Echelle logarithmique)

24

23

C'

C

SC

# FIG_4

30

34

30

32

31

33

31

35

36

FIG_5

FIG_6